# EUROPÄISCHE PATENTSCHRIFT

(11) **EP 1 863 630 B1**
(45) Veröffentlichungstag und Bekanntmachung des Hinweises auf die Patenterteilung: **19.03.2014**
(21) Anmeldenummer: 06725248.6
(22) Anmeldetag: 22.03.2006
(51) Int. Cl.: B30B 11/02, A61J 3/10

(54) **Verfahren und Vorrichtung zur Ultraschallverpressung einer Tablette oder einer multipartikulären Arzneiform**
Method and device for ultrasound-pressing a tablet or a multiparticulate medicament
Procédé et dispositif de compression par ultrasons d'un comprimé ou d'une forme galénique multiparticulaire

(30) Priorität: 22.03.2005 DE 102005013725; 23.05.2005 DE 102005024171
(43) Veröffentlichungstag der Anmeldung: 12.12.2007
(73) Patentinhaber: GRUENENTHAL GMBH, 52078 Aachen (DE)
(72) Erfinder: ARKENAU-MARIC, Elisabeth, Dr., 50931 Köln (DE); BARTHOLOMÄUS, Johannes, Dr., 52080 Aachen (DE); GEISSLER, Anja, Dr., 52222 Stolberg (DE); JANSEN, Rene, 52249 Eschweiler (DE)
(74) Vertreter: Wolff, Felix
(86) Internationale Anmeldenummer: PCT/EP2006/060965
(87) Internationale Veröffentlichungsnummer: WO 2006/100274

(56) Entgegenhaltungen:
- WO-A-01/21388
- CH-A- 486 317
- PATENT ABSTRACTS OF JAPAN Bd. 013, Nr. 095 (C-573), 6. März 1989 (1989-03-06) & JP 63 275511 A (KOBAYASHI KOOC:KK), 14. November 1988 (1988-11-14)
- PATENT ABSTRACTS OF JAPAN Bd. 018, Nr. 592 (M-1702), 11. November 1994 (1994-11-11) & JP 06 220504 A (HITACHI METALS LTD), 9. August 1994 (1994-08-09)

## Beschreibung

Die vorliegende Erfindung betrifft ein Verfahren und eine Vorrichtung zur Formgebung einer pharmazeutisch wirksamen Substanz, insbesondere Arzneimittel in Form von Pulvern, Granulaten oder multipartikulären Arzneiformen, wie z.B. Pellets und Mikrokapseln, für die Herstellung einer Tablette oder einer multipartikulären Arzneiform mittels Ultraschall, die einen Ultraschallgenerator, eine Sonotrode und eine Matrize, in die die pharmazeutisch wirksame Substanz einfüllbar ist, aufweist.

Derartige Ultraschalleinrichtungen zum Aufschmelzen und Verpressen von Tabletten sind aus dem Stand der Technik bekannt und beispielsweise in dem Artikel "Principles and Application of Ultrasound in pharmaceutical powder compression", Pharmaceutical research, Vol. 17,No. 3, 2000, Seite 257 - 265 beschrieben. Dieser Artikel wird hiermit als Referenz eingeführt. Die im Stand der Technik beschriebene Tablettierung mittels Ultraschall hat jedoch den Nachteil, dass beim Einsatz von Ultraschall die Sonotrode, der Unterstempel und/oder die Matrize einem sehr hohen Verschleiß unterliegen und/oder dass der Unterstempel und/oder die Matrize und/oder Teile der Ultraschalleinrichtung punktförmig zusammenschweißen. Um dies zu vermeiden, wird vom Stand der Technik beispielsweise vorgeschlagen, die Teile durch Folien voneinander zu trennen. Dies hat sich in der Praxis jedoch als sehr aufwändig, nicht handhabbar und kostenintensiv herausgestellt.

Ferner ist aus der Druckschrift JP 06,220504,A eine Maschine zum Verpressen von Pulver bekannt, bei welcher ein oberer und ein unterer Stempel in eine mit Pulver gefüllte Matrize eingefahren werden, wobei die Matrize mit einem Ultraschallgenerator versehen ist. Nachteilig an dieser Lösung ist, dass die Stempel beim Verpressen des Pulvers in direkten Kontakt mit der Matrize stehen und somit die Gefahr einer Verschweißung der Stempel mit der Matrize besteht.

Es war deshalb die Aufgabe der vorliegenden Erfindung, eine Vorrichtung zur Formgebung einer pharmazeutisch wirksamen Substanz, insbesondere Arzneimittel in Form von Pulvern, Granulaten oder multipartikulären Arzneiformen, wie z.B. Pellets und Mikrokapseln, für die Herstellung einer Tablette oder einer multipartikulären Arzneiform mittels Ultraschall zur Verfügung zu stellen, die die Nachteile des Standes der Technik nicht aufweist.

Gelöst wird die Aufgabe mit einer Vorrichtung, zur Formgebung einer pharmazeutisch wirksamen Substanz, insbesondere Arzneimittel in Form von Pulvern, Granulaten oder multipartikulären Arzneiformen, wie z.B. Pellets und Mikrokapseln, für die Herstellung einer Tablette oder einer multipartikulären Arzneiform mittels Ultraschall, mit einem Ultraschallgenerator, einer Sonotrode und einer Matrize, in die die pharmazeutisch wirksame Substanz einfüllbar ist, wobei die Sonotrode bei der Formgebung in direktem Kontakt zu der pharmazeutisch wirksamen Substanz steht, wobei die Sonotrode nicht in die Matrize eindringt und kein direkter Kontakt zwischen Sonotrode und Matrize besteht.

Es war für den Fachmann überaus erstaunlich und nicht zu erwarten, dass die erfindungsgemäße Vorrichtung einfach und kostengünstig herzustellen und zu betreiben ist. Die Standzeiten der Sonotorode und der Matrize sind deutlich erhöht. Mit der erfindungsgemäßen Vorrichtung kann eine Tablette oder eine multipartikuläre Arzneiform hergestellt werden, bei der ein Missbrauch einer solchen Tablette, die beispielsweise psychotrope Stoffe enthält, zumindest deutlich erschwert wird. Eine solchermaßen harte Tablette dient auch zum Vermeiden des Fehlgebrauchs von Arzneiformen mit hochaktiven Substanzen, denn solche Tabletten können aufgrund ihrer Härte nicht mittels eines Zerkleinerers, beispielsweise eines Mörsers, zerkleinert werden. Durch eine solche Zerkleinerung bzw. allgemein Zerstörung der mechanischen Struktur der Tablette kann nämlich die pharmakologische Wirksamkeit, insbesondere der zeitliche Verlauf der Abgabe der hochaktiven Substanz, insbesondere bei einer sogenannten Retard-Formulierung stark beeinträchtigt werden. Bei der Herstellung einer Tablette oder einer multipartikulären Arzneiform mittels der erfindungsgemäßen Vorrichtung werden keine Folien oder andere Trennmittel zwischen der Sonotrode und der zu komprimierenden pharmazeutischen Substanz benötigt.

Die erfindungsgemäße Vorrichtung eignet sich zur Formgebung einer pharmazeutisch wirksamen Substanz, insbesondere zur Formgebung von Pulvern, Granulaten, multipartikulären Arzneiformen, wie z.B. Pellets und Mikrokapseln, für die Herstellung einer Tablette oder einer multipartikulären Arzneiform unter dem Einsatz von Ultraschall. Formgebung im Sinne der Erfindung bedeutet, dass pharmazeutisch wirksame Substanzen als Ausgangssubstanzen, insbesondere als pulverförmige Arzneimittel, in die gewünschte Form gebracht und dabei zumindest teilweise kompaktiert werden. Durch den Einsatz von Ultraschall werden die Ausgangssubstanzen, d.h. insbesondere pulverförmige Arzneimittel, erwärmt und schmelzen dabei zumindest teilweise auf. Die so geschmolzenen Ausgangssubstanzen bilden einen Verbund. Unter einem gewissen Druck kann eine Komprimierung und eine zusätzliche Härtung der resultierenden Tablette oder der resultierenden multipartikulären Arneiform erfolgen. Pharmazeutisch wirksame Substanz im Sinne der Erfindung ist eine Substanz, die zum einen die gewünschte pharmazeutische Wirkung aufweist und zum anderen für die Formgebung mittels der erfindungsgemäßen Vorrichtung geeignet ist. Dies bedeutet, dass die pharmazeutisch wirksame Substanz erfindungsgemäß durchaus eine Mehrzahl von Stoffen umfassen kann (und in der Regel auch umfassen wird), von denen ein Teil vorwiegend oder ausschließlich die pharmazeutische Wirksamkeit aufweist und von denen ein anderer Teil in der erfindungsgemäßen Vorrichtung derart aufschmelzbar und miteinander verschweißbar bzw. sinterbar ist, dass dadurch ein Verbund bzw. eine Matrix zum weitgehenden mechanischen Einschluss des pharmazeutisch wirksamen Teils der Stoffe entsteht.

Weiterhin erfindungsgemäß weist die erfindungsgemäße Vorrichtung einen Ultraschallgenerator auf, der die Ultraschallfrequenzen, die sich vorzugsweise in einer Bandbreite zwischen 10 und 70 kHz, besonders bevorzugt zwischen 10 und 40 kHz bewegen, erzeugt. Diese Ultraschallfrequenzen werden gegebenenfalls verstärkt und dann an eine sogenannte Sonotrode übertragen, die im Ultraschallbereich schwingt und die Schwingungsenergie an die zu formenden Ausgangssubstanzen abgibt. Die Sonotrode ist vorzugsweise aus Edelstahl, Titan, gehärtetem Titan oder aus einer Legierung, die mindestens eine der genannten Substanzen enthält, gefertigt.

Weiterhin erfindungsgemäß weist die erfindungsgemäße Vorrichtung eine Matrize auf, in die die pharmazeutisch wirksame Substanz (zeitlich vor ihrer Formgebung) eingefüllt wird, wobei die Matrize zumindest teilweise das Negativ der zu erzielenden Tablettenform darstellt. Die Matrize ist vorzugsweise aus einem Material gefertigt, das beim Einkoppeln von Ultraschall keine Verbindung mit der zu tablettierenden Mischung, d.h. mit der zur Komprimierung vorgesehenen pharmazeutisch wirksamen Substanz, eingeht und das während des Schweiß- bzw. Formgebungsprozesses nicht reißt oder zerspringt (wie dies etwa bei Glas der Fall wäre). Vorzugsweise ist die Matrize mit solchen Eigenschaften aus Keramik, Edelstahl, Titan, hochschmelzendem Kunststoff und/oder gehärtetem Titan gefertigt.

Erfindungsgemäß ist die erfindungsgemäße Vorrichtung nun so gestaltet, dass die Sonotrode bei der Formgebung zwar in direktem Kontakt zu der pharmazeutisch wirksamen Substanz steht, es jedoch zu keinem Zeitpunkt der Formgebung zu einem direkten Kontakt zwischen der Matrize und der Sonotrode kommt. Dadurch ist zum einen die gewünschte Tablette bzw. die gewünschte multipartikuläre Arzneiform mit der gewünschten Form und der gewünschten Härte erzielbar, zum anderen tritt jedoch die o. g. Punktverschweißung nicht ein.

Vorzugsweise besteht ein Spalt zwischen der Sonotrode und der Matrize, dessen maximale Größe so bemessen ist, dass durch ihn kein Anteil der pharmazeutisch wirksamen Substanz, insbesondere zu Beginn der Ultraschallformgebung, entweichen kann. Vorzugsweise hat der Spalt eine Größe von weniger als 2 mm, bevorzugt weniger als 1 mm, besonders bevorzugt weniger als 0,7 mm.

In einer weiteren bevorzugten Ausführungsform ist zwischen der Sonotrode und der Matrize ein Abstandshalter angeordnet. Diese bevorzugte Ausführungsform der vorliegenden Erfindung ist insbesondere dann von Vorteil, wenn die Sonotrode während der Formgebung der pharmazeutisch wirksamen Substanz bewegt wird, beispielsweise um sicherzustellen, dass auch bei sich während der Formgebung verdichtender pharmazeutisch wirksamer Substanz immer ein guter Kontakt zwischen der pharmazeutisch wirksamen Substanz und der Sonotrode besteht und/oder um die ultraschallunterstützte Formgebung unter Druck durchführen zu können. Bei dieser Ausführungsform ist die Gefahr gegeben, dass die Sonotrode in die Matrize eindringt und dadurch ein direkter Kontakt zwischen der Sonotrode und der Matrize hergestellt wird, der zu einer Punktverschweißung und/oder zur Zerstörung der Sonotrode führen würde. Diese Kontaktbildung wird durch den Abstandstandhalter, der für einen konstanten Abstand zwischen Sonotrode und Matrize sorgt, verhindert.

Vorzugsweise ist, wie bereits erwähnt, die Sonotrode axial verschieblich. Diese bevorzugte Ausführungsform der vorliegenden Erfindung ist insbesondere dann von Vorteil, wenn die Sonotrode einer gewissen Druckbelastung ausgesetzt ist und sich die pharmazeutisch wirksame Substanz bei der Formgebung komprimiert.

Weiterhin bevorzugt ist die Matrize ebenfalls verschieblich gelagert bzw. weist die Matrize eine Vorweite auf.

Ganz besonders bevorzugt ist die Bewegung der Sonotrode und der Matrize nach Einstellung des Spaltes gleichförmig. Dadurch wird erreicht, dass sich der Spalt weder verkleinert noch vergrößert, so dass weder pulverförmiges oder geschmolzenes Arzneimittelpulver - d.h. Anteile der pharmazeutisch wirksamen Substanz vor ihrer endgültigen Formgebung - aus dem Spalt austreten können, noch die Sonotrode zerstört wird und/oder die Matrize mit dem Unterstempel punktförmig miteinander verschweißen.

In einer weiteren bevorzugten Ausführungsform weist die erfindungsgemäße Vorrichtung einen Stempel auf, der sich auf der der Sonotrode gegenüberliegenden Seite der Matrize befindet. In diesem Fall ist die Matrize vorzugsweise lediglich ringförmig gestaltet und das Volumen, in welches die pharmazeutisch wirksame Substanz vor ihrer erfindungsgemäßen Formgebung eingefüllt wird, wird von unten durch einen Stempel begrenzt. Vorzugsweise ist dieser Stempel so gelagert, dass beim Einkoppeln des Ultraschalls in die pharmazeutisch wirksame Substanz der Stempel nicht oder nur so weit mitschwingt, dass ein Stoffschluss zwischen dem Stempel und der Matrize ausgeschlossen werden kann. Dies wird vorzugsweise dadurch erreicht, dass der Stempel in einem schallharten Bauteil vorzugsweise form- und/oder kraftschlüssig gelagert ist. Vorzugsweise ist der Stempel axial verschieblich gelagert. Diese Ausführungsform hat insbesondere den Vorteil, dass die Sonotrode bei der Ultraschallformgebung feststehend gestaltet werden kann und der ggf. für die Formgebung benötigte Druck durch den Stempel in die pharmazeutisch wirksame Substanz eingebracht werden kann. Des weiteren ist der Stempel vorzugsweise so gelagert, dass seine Eindringtiefe in die Matrize einstellbar ist. Diese Ausführungsform hat insbesondere den Vorteil, dass das Volumen bzw. die Menge der pharmazeutisch wirksamen Substanz, das bzw. die in die Matrize eingefüllt und dann der Formgebung ausgesetzt wird, einstellbar ist.

Wie bereits erwähnt, ist es in der Regel von Vorteil, wenn die Formgebung mittels Ultraschall und unter Einwirkung einer gewissen Kraft während der Ultraschallkopplung erfolgt, wobei die Kraft vorzugsweise bis 10 kN beträgt, besonders bevorzugt bis 5 kN und ganz besonders bevorzugt bis 2 kN. Diese Kraft kann durch die Sonotrode und/oder durch einen Stempel auf das pulverförmige Arzneimittel, d.h. die pharmazeutisch wirksame Substanz, übertragen werden.

Die Dauer der Ultraschallbehandlung bzw. der Temperaturverlauf hängt insbesondere von dem gewünschten Resultat ab und wird von dem Fachmann experimentell ermittelt.

Ein weiterer Gegenstand der vorliegenden Erfindung ist ein Verfahren zur Formgebung einer pharmazeutisch wirksamen Substanz, insbesondere Arzneimittel in Form von Pulvern, Granulaten oder multipartikulären Arzneiformen, wie z.B. Pellets und Mikrokapseln, für die Herstellung einer Tablette oder einer multipartikulären Arzneiform mittels Ultraschall, mit einem Ultraschallgenerator, einer Sonotrode und einer Matrize, in die die pharmazeutisch wirksame Substanz einfüllbar ist, bei dem die Sonotrode und die Matrize bei der Formgebung so bewegt werden, dass immer ein Spalt zwischen Sonotrode und Matrize aufrecht erhalten bleibt und die Sonotrode bei der Formgebung in direktem Kontakt zu der zu plastifizierenden bzw. zu komprimierenden pharmazeutisch wirksamen Substanz, vorzugsweise Arzneimittel, steht.

Ein weiterer Gegenstand der vorliegenden Erfindung ist ein Verfahren zur Formgebung einer pharmazeutisch wirksamen Substanz, insbesondere Arzneimittel in Form von Pulvern, Granulaten oder multipartikulären Arzneiformen, wie z.B. Pellets und Mikrokapseln, für die Herstellung einer Tablette oder einer multipartikulären Arzneiform mittels Ultraschall mit einem Ultraschallgenerator, einer Sonotrode und einer Matrize, in die die pharmazeutisch wirksame Substanz, insbesondere das pulverförmige Arzneimittel, einfüllbar ist, wobei bei dem Verfahren die Sonotrode beim Schweißen in direktem Kontakt zu dem Arzneimittel, d.h. der pharmazeutisch wirksamen Substanz, steht und ein Spalt zwischen Sonotrode und Matrize besteht und dass beim Schweißen ein Stempel in Richtung der Sonotrode bewegt wird.

Beide erfindungsgemäße Verfahren sind einfach und kostengünstig durchzuführen und eignen sich zur Herstellung von Tabletten mit hoher Härte, die beispielsweise psychotrope Substanzen aufweisen und bei denen ein Fehlgebrauch oder ein einfacher Missbrauch ausgeschlossen werden kann.

Ein weiterer Gegenstand der vorliegenden Erfindung ist deshalb die Verwendung der erfindungsgemäßen Vorrichtung und/oder das erfindungsgemäße Verfahren zur Herstellung von solchen Arzneimitteln, insbesondere Tabletten oder multipartikuläre Arzneiformen, die psychotrope Stoffe enthalten und die nicht betäubungsmittelverschreibungspflichtig sind, bzw. die eine große Härte aufweisen, so dass ein Fehlgebrauch zuverlässig vermieden wird.

Alle oben gemachten Ausführungen gelten für die erfindungsgemäße Vorrichtung und die erfindungsgemäßen Verfahren und die erfindungsgemäße Verwendung gleichermaßen.

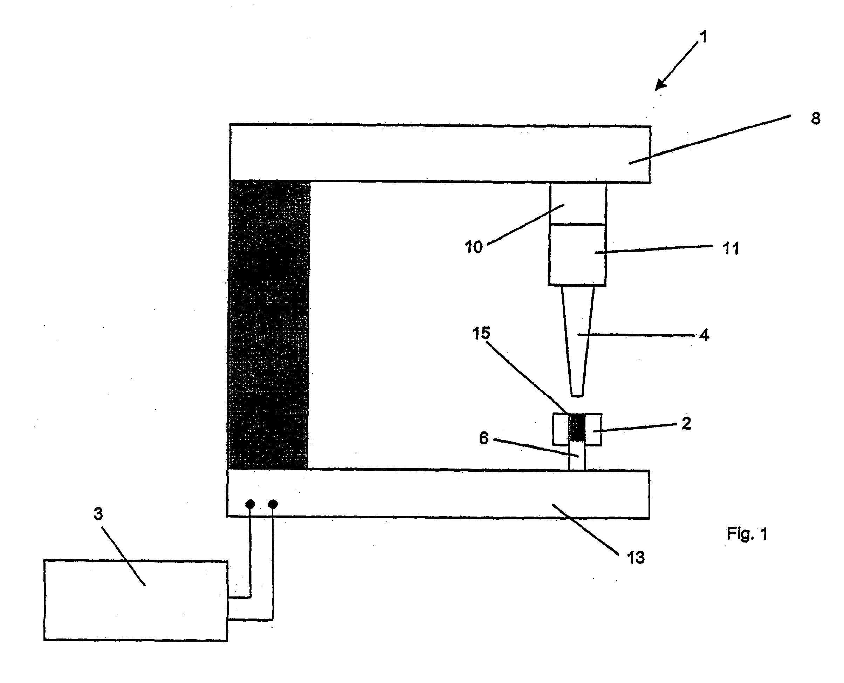

Im Folgenden wird die Erfindung anhand der **Figuren 1 - 5** erläutert. Diese Erläuterungen sind lediglich beispielhaft und schränken den allgemeinen Erfindungsgedanken nicht ein. Die Erläuterungen gelten für die erfindungsgemäße Vorrichtung, die erfindungsgemäßen Verfahren und die erfindungsgemäße Verwendung gleichermaßen.
**Figur 1** zeigt eine Prinzipskizze der erfindungsgemäßen Vorrichtung.
**Figur 2** zeigt eine Detaildarstellung der erfindungsgemäßen Vorrichtung insbesondere der Formgebung.
**Figur 3** zeigt ein Detail der erfindungsgemäßen Vorrichtung während der Formgebung.
**Figur 4** zeigt die Darstellung gemäß Figur 3 nach Fertigstellung der Tablette.
**Figur 5** zeigt eine weitere Ausführungsform der erfindungsgemäßen Vorrichtung.
**Figur 6** zeigt eine alternative Ausführungsform der erfindungsgemäßen Vorrichtung mit einer Vorweite im oberen Bereich der Matrize.

**Figur 1** zeigt eine Ausführungsform der erfindungsgemäßen Vorrichtung 1. Die Vorrichtung 1 weist eine Sonotrode 4 auf, die mit einem Verstärker 11 verbunden ist, der wiederum von einem Konverter 10, beispielsweise einem Piezoelement, das elektrische Signale von dem Generator 3 in Schwingungen umwandelt, die von dem Verstärker 11 verstärkt und and die Sonotrode 4 weitergeleitet werden. Des weiteren weist die Vorrichtung 1 eine Presse 8 auf, mit der die Sonotrode 4 mit einem gewissen Druck vertikal nach unten gedrückt werden kann. Des weiteren weist die erfindungsgemäße Vorrichtung eine Matrize 2 auf, die mit einem Stempel 6 in Verbindung steht. Durch die Matrize 2 und den Stempel 6 wird ein Innenraum definiert, der mit einer pharmazeutisch wirksamen Substanz 15 füllbar ist. Der Stempel 6 ist in einer schallharten Basisplatte 13 so gelagert, dass er beim Einkoppeln des Ultraschalls in die pharmazeutisch wirksame Substanz 15 nicht oder nur so mitschwingt, dass es zu keinen Punktverschweißungen zwischen der Matrize 2 und dem Stempel 6 kommt. Während des Schweiß- bzw. Sintervorgangs der pharmazeutisch wirksamen Substanz, während dem erfindungsgemäß gleichzeitig die Formgebung erfolgt, wird der Unterstempel auf einer Position gehalten, die zur Durchführung des erfindungsgemäßen Verfahrens geeignet ist, insbesondere derart, dass der Stempel und die Matrize nicht zusammenschweißen bzw. dass die Tablette das gewünschte Volumen hat. Die Kraft zum Verdichten des Produkts wird über die Sonotrode während und gegebenenfalls auch nach dem Schweißvorgang aufgebracht.

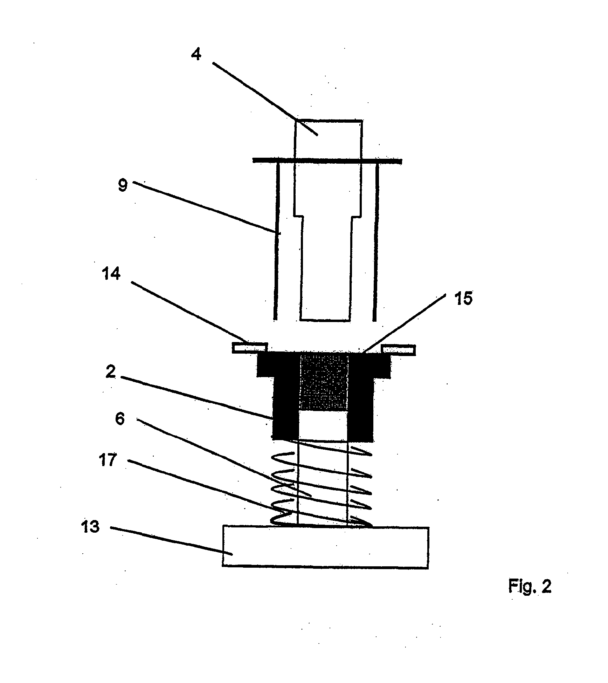

**Figur 2** zeigt die Stempel-Matrize-Sonotrodenanordnung im Detail. Es ist zu erkennen, dass an der Sonotrode 4 ein Abstandshalter 9 angebracht ist, der sich gemeinsam mit der Sonotrode 4 auf- und ab bewegt. Des weiteren ist zu erkennen, dass die Matrize 2 relativ zu dem Stempel 6 verschieblich gelagert ist und dass zwischen der Matrize 2 und der Basisplatte 13 eine Feder 17 (auch als Kraftgeber bezeichnet) angeordnet ist, die die Matrize 2 nach oben gegen ein Begrenzungsmittel 14 drückt. Der in Figur 2 dargestellte Zustand ist die Konfiguration vor dem Formen einer in Figur 2 nicht dargestellten Tablette 19. Der durch die Matrize 2 und den Stempel 6 definierte Innenraum ist mit der pharmazeutisch wirksamen Substanz 15 gefüllt. Die Lage des Stempels 6 kann relativ zu der Matrize 2 verändert werden, um das mit der pharmazeutisch wirksamen Substanz 15 zu füllende Volumen zu verändern. Dies kann beispielsweise dadurch erfolgen, dass in der Matrize 2 oder der Basisplatte 13 ein Gewinde angeordnet ist, das mit einem Gewinde in dem Stempel 6 korrespondiert. Durch Drehen des Stempels 6 kann das für das Arzneimittelpulver zur Verfügung stehende Volumen verändert werden. Die Sonotrode 4 ist in dem vorliegenden Fall aus gehärtetem Titan gefertigt. Die Matrize 2 kann entweder aus Edelstahl, Titan, gehärtetem Titan, hochschmelzendem Kunststoff oder Keramik gefertigt sein.

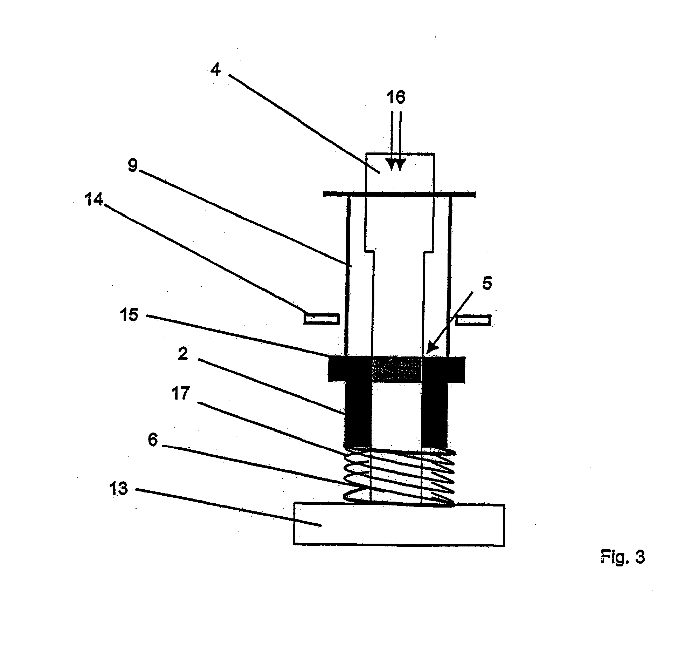

**Figur 3** zeigt die Formgebung einer Tablette. Durch die Schwingungen der Sonotrode 4 im Ultraschallfrequenzbereich und durch den mit dem Doppelpfeil dargestellten Druck 16, der auf die Sonotrode 4 wirkt, wird das Volumen der pharmazeutisch wirksamen Substanz 15 komprimiert, so dass die Sonotrode 4 sich während der Formgebung, d. h. während des Komprimierens und Schweißens der pharmazeutisch wirksamen Substanz 15 zur Herstellung der Tablette nach unten bewegt. Der Abstandshalter 9, der mit der Sonotrode 4 fest verbunden ist und dessen unteres Ende mit der Matrize 2 zusammenwirkt, drückt diese bei der Abwärtsbewegung der Sonotrode 4 ebenfalls mit nach unten, so dass der Ringspalt 5 (auch als axialer Spalt bezeichnet), der sich zwischen dem unteren Ende der Sonotrode 4 und der Matrize 2 befindet und der durch den Abstandshalter 9 einstellbar ist, während des gesamten Formgebungsvorganges nicht verändert. Der Spalt 5 wird so eingestellt, dass die Sonotrode 4 die Matrize 2 nicht berührt, dass jedoch sichergestellt ist, dass während der Formgebung keine pharmazeutisch wirksame Substanz, sei es in ihrer ursprünglichen Form (Pulver, Granulat, multipartikulär) oder in einer intermediären Form während der Durchführung des erfindungsgemäßen Verfahrens (beispielsweise in geschmolzener Form) aus der erfindungsgemäßen Vorrichtung 1 entweicht und dass die Sonotrode 4 sich im ständigen Kontakt zu der zu verarbeitenden pharmazeutisch wirksamen Substanz 15 befindet. Durch die Abwärtsbewegung der Matrize 2 wird die Feder 17 komprimiert.

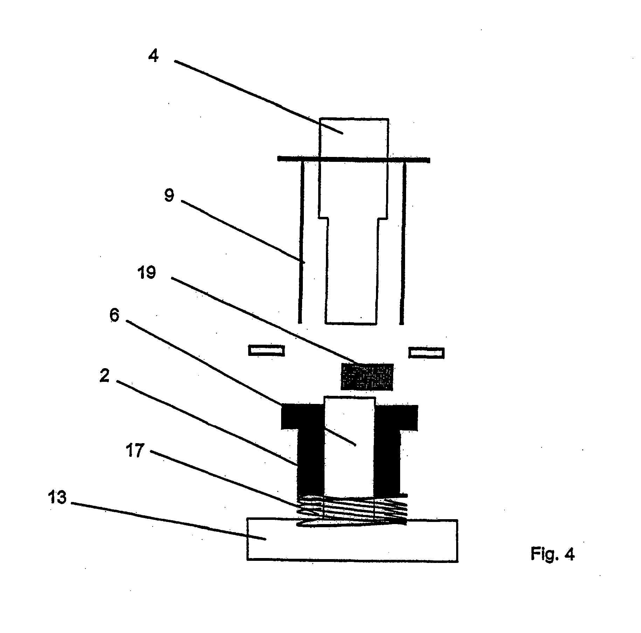

Figur 4 zeigt den Auswurf der Tablette 19. Nachdem die Tablette 19 fertiggestellt worden ist, wird die Matrize 2 noch weiter nach unten geschoben, bis ihre Oberkante zumindest auf gleicher Höhe mit der Oberkante des Stempels 6 ist und in dieser Position für einen kurzen Augenblick festgehalten. Gleichzeitig wird die Sonotrode 4 und damit der Abstandshalter 9 nach oben bewegt. Die Tablette 19 liegt nunmehr nur noch auf dem Stempel 6 auf und kann dadurch leicht entnommen werden. Sobald die Tablette 19 entfernt worden ist, schnellt die Matrize 2 wieder nach oben, bis sie gegen den Wegbegrenzer 14 läuft und das so gebildete Volumen kann erneut mit pharmazeutisch wirksamer Substanz 15 gefüllt werden, wonach diese neue Füllung von pharmazeutisch wirksamer Substanz 15 - wie es gemäß den Figuren 2 und 3 beschrieben worden ist - ebenfalls komprimiert und verschweißt werden kann. Der Fachmann erkennt, dass die Entformung der Tablette 19 aus der Matrize auch durch ein Anheben des Stempels 6 erfolgen kann.

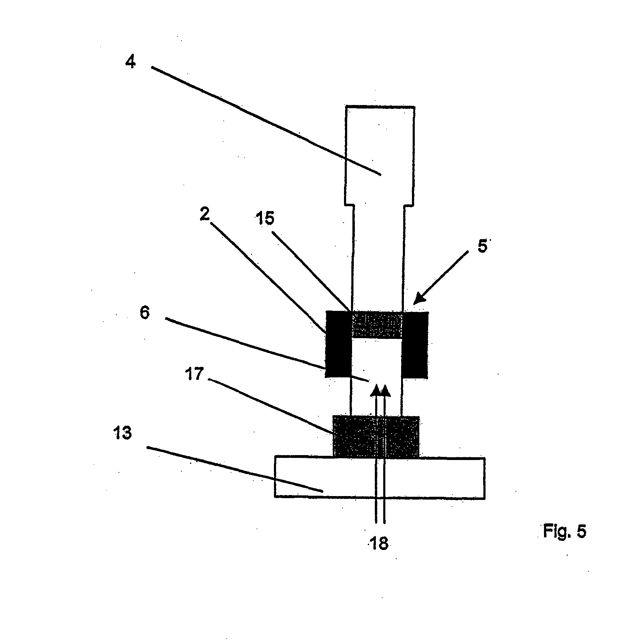

**Figur 5** zeigt eine andere Ausführungsform der erfindungsgemäßen Vorrichtung. In diesem Fall ist die Sonotrode 4 während der Formgebung so ortsfest angeordnet, dass ein Ringspalt 5 (auch als axialer Spalt bezeichnet) zwischen Sonotrode 4 und Matrize 2 vorhanden ist, der den oben genannten Kriterien genügt. Der Volumenverlust der pharmazeutisch wirksamen Substanz während der Formgebung wird in dem vorliegenden Fall durch den vertikal beweglichen Stempel 6 kompensiert, der durch das Druckmittel 17 (auch als Kraftgeber bezeichnet) mit Kraft beaufschlagt wird und der sich während des Formgebungsvorganges nach oben bewegt. Auch dieser Stempel 6 ist so an der schallharten Basisplatte13 bzw. an dem schallharten Druckmittel 17 angeordnet, dass er nicht oder nur so schwingt, dass keine Punktverschweißung zwischen dem Stempel 6 und der Matrize 2 erfolgen kann. Die Entformung und Entnahme der fertiggestellten Tablette erfolgt im Wesentlichen wie in Figur 4 dargestellt.

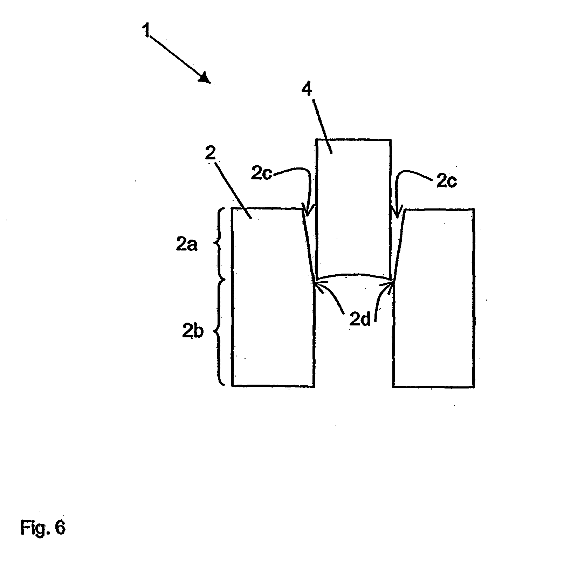

In **Figur 6** ist eine alternative Ausführungsform der erfindungsgemäßen Vorrichtung 1 mit einer Vorweite 2c im oberen Bereich 2a der Matrize 2 dargestellt. Die Matrize 2 ist in Figur 6 in einer Schnittdarstellung abgebildet. Die Matrize 2 ist im unteren Bereich 2b zylindrisch vorgesehen. Im Bereich der Vorweite 2c ist die Matrize 2 zur Sonotrode 4 hin sich weitend, insbesondere konisch geformt, vorgesehen. Durch diese Formgebung ist es erfindungsgemäß besonders effizient möglich, die in Figur 6 nicht dargestellte pharmazeutisch wirksame Substanz 15 vor ihrem Verschmelzen bzw. Verschweißen zu verdichten. Weiterhin ist es durch diese Formgebung der Matrize 2 besonders wirkungsvoll möglich, Lufteinschlüsse innerhalb der pharmazeutisch wirksamen Substanz und daher auch innerhalb der ebenfalls nicht dargestellten fertigen Tablette 19 zu verhindern. Bei der Durchführung des erfindungsgemäßen Verfahrens zur Formgebung fährt die Sonotrode 4 bis kurz vor den Übergang zwischen dem unteren, vorzugsweise zylindrischen, Bereich 2b der Matrize 2 und dem oberen Bereich 2a, welcher der Vorweite 2c entspricht. Erfindungsgemäß ist es bevorzugt vorgesehen, dass die Sonotrode 4 zu diesem Übergang zwischen den Bereichen 2a und 2b, der beispielsweise als eine Kante 2d ausgeführt sein kann, in jedem Stadium des Verfahrens zur Formgebung einen Abstand einhält, der bevorzugt kleiner als 1 mm, besonders bevorzugt kleiner als 0,7 mm ist. Die Vorweite 2c muss so gewählt werden, dass die Sonotrode 4 beim Einkoppeln von Ultraschall nicht die Matrize 2 berührt.

### Bezugszeichenliste

- 1: Vorrichtung
- 2: Matrize
- 2a: oberer Bereich der Matrize
- 2b: unterer Bereich der Matrize
- 2c: Vorweite
- 2d: Kante zwischen oberem und unterem Bereich der Matrize
- 3: Ultraschallgenerator
- 4: Sonotrode
- 5: Axialer Spalt
- 6: Stempel
- 8: Druckmittel
- 9: Abstandshalter
- 10: Konverter
- 11: Verstärker, Booster
- 13: schallharte Basisplatte
- 14: obere Wegbegrenzung für die Matrize
- 15: pharmazeutisch wirksame Substanz, insbesondere Arzneimittelpulver
- 16: Druck, Axialbewegung der Sonotrode
- 17: Kraftgeber (pneumatisch oder hydraulisch betrieben)
- 18: axiale Längsverschiebung des Stempels 6
- 19: fertiggestellte Tablette

## Patentansprüche

1. Vorrichtung (1), zur Formgebung einer pharmazeutisch wirksamen Substanz (15) in Form von Pulvern, Granulaten, multipartikulären Arzneiformen, insbesondere Pellets und Mikrokapseln, für die Herstellung einer Tablette (19) oder einer multipartikulären Arzneiform mittels Ultraschall, mit einem Ultraschallgenerator (3), einer Sonotrode (4) und einer Matrize (2), in die die pharmazeutisch wirksame Substanz (15) einfüllbar ist, wobei die Sonotrode (4) bei der Formgebung in direktem Kontakt zu der pharmazeutisch wirksamen Substanz (15) steht, **dadurch gekennzeichnet, dass** die Sonotrode (4) nicht in die Matrize (2) eindringt und kein direkter Kontakt zwischen Sonotrode (4) und Matrize (2) besteht.

2. Vorrichtung (1) nach Anspruch 1, **dadurch gekennzeichnet, dass** sich ein Spalt (5) zwischen der Sonotrode (4) und der Matrize (2) befindet, dessen maximale Größe so bemessen ist, dass durch ihn bei keine pharmazeutisch wirksame Substanz (15) entweicht.

3. Vorrichtung (1) nach einem der voranstehenden Ansprüche, **dadurch gekennzeichnet, dass** zwischen der Sonotrode (4) und der Matrize (2) ein Abstandshalter (9) angeordnet ist.

4. Vorrichtung (1) nach einem der voranstehenden Ansprüche, **dadurch gekennzeichnet, dass** die Sonotrode (4) axial verschieblich angeordnet ist.

5. Vorrichtung (1) nach einem der voranstehenden Ansprüche, **dadurch gekennzeichnet, dass** die Matrize (2) verschieblich gelagert ist.

6. Vorrichtung (1) nach einem der voranstehenden Ansprüche, **dadurch gekennzeichnet, dass** die Matrize (2) eine Vorweite (2c) aufweist.

7. Vorrichtung (1) nach einem der voranstehenden Ansprüche, **dadurch gekennzeichnet, dass** die Bewegungen der Sonotrode (4) und der Matrize (2) nach Einstellung des Spaltes (5) gleichförmig erfolgt.

8. Vorrichtung (1) nach einem der voranstehenden Ansprüche, **dadurch gekennzeichnet, dass** sie einen Stempel (6) aufweist, der sich auf der der Sonotrode (4) gegenüberliegenden Seite der Matrize (2) befindet.

9. Vorrichtung (1) nach Anspruch 8, **dadurch gekennzeichnet, dass** der Stempel (6) so gelagert ist, dass beim Einkoppeln des Ultraschalls der Stempel (6) nicht mitschwingt.

10. Vorrichtung (1) nach einem der voranstehenden Ansprüche, **dadurch gekennzeichnet, dass** der Stempel (6) beweglich angeordnet ist.

11. Vorrichtung (1) nach einem der voranstehenden Ansprüche, **dadurch gekennzeichnet, dass** sie ein Druckmittel (8) aufweist, das auf die Sonotrode (4) und/oder auf den Stempel (6) einwirkt und die Formgebung der Tablette (19) oder der multipartikulären Arzneiform unterstützt.

12. Verfahren zur Formgebung einer pharmazeutisch wirksamen Substanz (15), insbesondere Arzneimittel in Form von Pulvern, Granulaten, multipartikulären Arzneiformen, wie z.B. Pellets und Mikrokapseln, für die Herstellung einer Tablette (19) oder einer multipartikulären Arzneiform mittels Ultraschall, mit einem Ultraschallgenerator (3), einer Sonotrode (4) und einer Matrize (2), in die die pharmazeutisch wirksame Substanz (15) einfüllbar ist, wobei die Sonotrode (4) bei der Formgebung in direktem Kontakt zu der zu plastifizierenden pharmazeutisch wirksamen Substanz (15) steht, **dadurch gekennzeichnet, dass** die Sonotrode (4) und die Matrize (2) bei der Formgebung so bewegt werden, dass immer ein axialer Spalt (5) zwischen Sonotrode (4) und Matrize (2) aufrecht erhalten bleibt.

13. Verfahren nach Anspruch 12, wobei in die Matrize (2) ein pulverförmiges Arzneimittel einfüllbar ist, wobei die Sonotrode (4) beim Schweißen in direktem Kontakt zu der pharmazeutisch wirksamen Substanz (15) steht und wobei die Sonotrode (4) nicht in die Matrize (2) eindringt und der Spalt (5) zwischen Sonotrode (4) und Matrize (2) besteht, wobei beim Schweißen ein Stempel (6) in Richtung der Sonotrode (4) bewegt wird.

14. Verwendung der Vorrichtung und/oder des Verfahrens nach einem der voranstehenden Ansprüche zur Herstellung von Arzneimitteln, die psychotrope Stoffe enthalten und die nicht betäubungsmittelverschreibungspflichtig sind.

## Claims

1. Device (1) for shaping a pharmaceutically active substance (15) in the form of powders, granules, multiparticulate medicaments, in particular pellets and microcapsules, for the production of a tablet (19) or a multiparticulate medicament by means of ultrasound, with an ultrasound generator (3), a sonotrode (4) and a mould (2), into which the pharmaceutically active substance can be poured (15), wherein, during the shaping, the sonotrode (4) is in direct contact with the pharmaceutically active substance (15), **characterised in that** the sonotrode (4) does not penetrate the mould (2) and there is no direct contact between the sonotrode (4) and the mould (2).

2. Device (1) according to claim 1, **characterised in that** there is a gap (5) between the sonotrode (4) and the mould (2) of which the maximum size is dimensioned so that no pharmaceutically active substance (15) escapes through it.

3. Device (1) according to any one of the preceding claims, **characterised in that** a spacer (9) is disposed between the sonotrode (4) and the mould (2).

4. Device (1) according to any one of the preceding claims, **characterised in that** the sonotrode (4) is disposed in an axially displaceable manner.

5. Device (1) according to any one of the preceding claims, **characterised in that** the mould (2) is displaceably mounted.

6. Device (1) according to any one of the preceding claims, **characterised in that** the mould (2) comprises a pre-widening (2c).

7. Device (1) according to any one of the preceding claims, **characterised in that** the movements of the sonotrode (4) and the mould (2) take place uniformly after the establishment of the gap (5).

8. Device (1) according to any one of the preceding claims, **characterised in that** it comprises a die (6) located on the side of the mould (2) opposite to the sonotrode (4).

9. Device (1) according to claim 8, **characterised in that** the die (6) is mounted in such a way that when the ultrasound is coupled-in, the die (6) does not co-oscillate.

10. Device (1) according to any one of the preceding claims, **characterised in that** the die (6) is disposed movably.

11. Device (1) according to any one of the preceding claims, **characterised in that** it comprises a pressure means (8) which acts on the sonotrode (4) and/or on the die (6) and supports the shaping of the tablet (19) or the multiparticulate medicament.

12. Method for shaping a pharmaceutically active substance (15), in particular a drug, in the form of powders, granules, multiparticulate medicaments, such as, for example, pellets and microcapsules, for the production of a tablet (19) or a multiparticulate medicament by means of ultrasound, with an ultrasound generator (3), a sonotrode (4) and a mould (2), into which the pharmaceutically active substance (15) can be poured, wherein, during the shaping, the sonotrode (4) is in direct contact with the pharmaceutically active substance (15) that is to be plasticised, **characterised in that** during the shaping the sonotrode (4) and the mould (2) are moved in such a way that an axial gap (5) is always maintained between the sonotrode (4) and the mould (2).

13. Method according to Claim 12, wherein a powdered drug can be poured into the mould (2), wherein, during the welding, the sonotrode (4) is in direct contact with the pharmaceutically active substance (15) and wherein the sonotrode (4) does not penetrate the mould (2) and there is the gap (5) between the sonotrode (4) and the mould (2), wherein, during the welding, a die (6) is moved in the direction of the sonotrode (4).

14. Use of the device and/or the method according to any one of the preceding claims for the production of drugs containing psychotropic substances and which are not subject to medical prescription as narcotic drugs.

## Revendications

1. Dispositif (1), destiné au façonnage d'une substance pharmaceutiquement active (15) sous forme de poudres, de granulats, de formes médicamenteuses multiparticulaires, en particulier de pellets et de microcapsules, pour la préparation d'un comprimé (19) ou d'une forme médicamenteuse multiparticulaire au moyen d'ultrasons, présentant un générateur d'ultrasons (3), une sonotrode (4) et une matrice (2), dans laquelle la substance pharmaceutiquement active (15) peut être transvasée, la sonotrode (4) étant en contact direct avec la substance pharmaceutiquement active (15) lors du façonnage, **caractérisé en ce que** la sonotrode (4) ne pénètre pas dans la matrice (2) et qu'il n'existe pas de contact direct entre la sonotrode (4) et la matrice (2).

2. Dispositif (1) selon la revendication 1, **caractérisé en ce qu'**il existe une fente (5) entre la sonotrode (4) et la matrice (2), dont la taille maximale est dimensionnée de manière telle qu'il n'y a pas de substance pharmaceutiquement active (15) qui s'échappe par celle-ci.

3. Dispositif (1) selon l'une quelconque des revendications précédentes, **caractérisé en ce qu'**un espaceur (9) est disposé entre la sonotrode (4) et la matrice (2).

4. Dispositif (1) selon l'une quelconque des revendications précédentes, **caractérisé en ce que** la sonotrode (4) est disposée de manière à pouvoir être déplacée axialement.

5. Dispositif (1) selon l'une quelconque des revendications précédentes, **caractérisé en ce que** la matrice (2) est logée de manière à pouvoir être déplacée.

6. Dispositif (1) selon l'une quelconque des revendications précédentes, **caractérisé en ce que** la matrice (2) présente une largeur avant (2c).

7. Dispositif (1) selon l'une quelconque des revendications précédentes, **caractérisé en ce que** les mouvements de la sonotrode (4) et de la matrice (2) sont réalisés de manière uniforme après le réglage de la fente (5).

8. Dispositif (1) selon l'une quelconque des revendications précédentes, **caractérisé en ce qu'**il présente un poinçon (6) qui se trouve sur la face de la matrice (2) opposée à la sonotrode (4).

9. Dispositif (1) selon la revendication 8, **caractérisé en ce que** le poinçon (6) est logé de manière telle que le poinçon (6) n'oscille pas conjointement lors du démarrage des ultrasons.

10. Dispositif (1) selon l'une quelconque des revendications précédentes, **caractérisé en ce que** le poinçon (6) est disposé de manière mobile.

11. Dispositif (1) selon l'une quelconque des revendications précédentes, **caractérisé en ce qu'**il présente un agent sous pression (8) qui agit sur la sonotrode (4) et/ou sur le poinçon (6) et qui soutient le façonnage du comprimé (19) ou de la forme médicamenteuse multiparticulaire.

12. Procédé destiné au façonnage d'une substance pharmaceutiquement active (15), en particulier d'un médicament sous forme de poudres, de granulats, de formes médicamenteuses multiparticulaires, comme par exemple les pellets et les microcapsules, pour la préparation d'un comprimé (19) ou d'une forme médicamenteuse multiparticulaire au moyen d'ultrasons, présentant un générateur d'ultrasons (3), une sonotrode (4) et une matrice (2), dans laquelle la substance pharmaceutiquement active (15) peut être transvasée, la sonotrode (4) étant en contact direct avec la substance pharmaceutiquement active (15) plastifiante lors du façonnage, **caractérisé en ce que** la sonotrode (4) et la matrice (2) sont déplacées lors du façonnage de manière telle qu'une fente axiale (5) est toujours maintenue entre la sonotrode (4) et la matrice (2).

13. Procédé selon la revendication 12, un médicament sous forme de poudre pouvant être transvasé dans la matrice (2), la sonotrode (4) étant en contact direct avec la substance pharmaceutiquement active (15) lors du soudage et la sonotrode (4) ne pénétrant pas dans la matrice (2) et la fente (5) existant entre la sonotrode (4) et la matrice (2), un poinçon (6) étant déplacé vers la sonotrode (4) lors du soudage.

14. Utilisation du dispositif et/ou du procédé selon l'une quelconque des revendications précédentes pour la fabrication de médicaments, qui contiennent des substances psychotropes et qui ne sont pas soumis à une prescription médicale de stupéfiants.
